# EUROPEAN PATENT APPLICATION

(11) **EP 3 441 047 A1**
(43) Date of publication of application: **13.02.2019**
(21) Application number: 18187590.7
(22) Date of filing: 06.08.2018
(51) Int. Cl.: A61F 5/445, A61F 2/00, A61B 17/11, A61B 17/34, A61M 39/02

(54) **STENT AND ASSOCIATED METHODOLOGIES FOR CREATING A STOMA**

(30) Priority: 07.08.2017 US 201762541958 P; 10.07.2018 US 201816030884
(71) Applicant: Covidien LP, Mansfield, MA 02048 (US)
(72) Inventor: AVVLN, Srinivasa Murthy Aravalli, 534211 Hyderabad (IN)
(74) Representative: Maschio, Antonio

(57) **Abstract**

A method for facilitating formation of a stoma includes positioning a stent within an opening extending through an abdominal wall of a subject, accessing an internal intestinal segment through the opening in the abdominal wall, passing the intestinal segment through the stent and external of the abdominal wall and securing end margins of the intestinal segment against the abdominal wall surrounding the opening.

## Description

### CROSS-REFERENCE TO RELATED APPLICATIONS

This application claims the benefit of and priority to U.S. Provisional Patent Application No. 62/541,958 filed August 7, 2017, the entire disclosure of which is incorporated by reference herein.

### TECHNICAL FIELD

The present disclosure generally relates to a surgical apparatus and associated method for establishing a stoma, and, in particular, relates to a stent for supporting tissue surrounding the stoma to minimize the potential of the formation of a parastomal herniation.

### BACKGROUND

Exteriorization of an internal body vessel such as the intestine is called a stoma. Stomas may be created in conjunction with an ostomy procedure by suturing a bisected portion of an intestine to the abdominal wall to provide internal access into the intestine for collecting fecal matter. Complications associated with stomas can include leaks, bleeding, necrosis, stenosis, retraction, dermal infection, mucocutaneous separation, prolapse, diversion colitis, etc. One significant complication is the potential development of a parastomal hernia. A parastomal hernia may occur when an intestinal section passes through the dissected area of fascia and muscle (e.g., anterior or posterior rectus sheath), which was needed to externalize the stoma, but has enlarged due, in-part, to intra-peritoneal forces and other tangential forces on the rectus sheath. As a consequence, the intestinal section herniates or bulges into the subcutaneous tissue adjacent the stoma, in effect, trapping the organs within the tissue and further complicating recovery.

### SUMMARY

Accordingly, the present disclosure is directed to a stent and associated method for implantation in connection with an ostomy procedure to maintain patency of the stoma and minimize the potential of complications including the formation of a parastomal hernia. In accordance with one exemplary embodiment, a method for facilitating formation of a stoma includes positioning a stent within an opening extending through an abdominal wall of a subject, accessing an internal intestinal segment through the opening in the abdominal wall, passing the intestinal segment through the stent and external of the abdominal wall and securing end margins of the intestinal segment against the abdominal wall surrounding the opening.

In embodiments, the method includes securing the stent to the abdominal wall. In some embodiments, the stent includes a stent body and at least one end flange disposed at an end of the stent body and wherein securing the stent includes attaching the end flange to the abdominal wall. In other embodiments, the stent includes end flanges at each end of the stent body and wherein securing the stent includes attaching each end flange to the abdominal wall. In embodiments, the end flanges each include anchors and wherein securing the stent includes penetrating the abdominal wall with the anchors of the end flanges.

In some embodiments, securing the stent includes penetrating at least one of the superficial fascia, deep fascia and visceral fascia with the anchors of the end flanges. In other embodiments, securing the stent includes penetrating at least two of the superficial fascia, deep fascia and visceral fascia with the anchors of the end flanges.

In embodiments, the method includes forming an incision within the abdominal wall with the incision being the opening.

In certain embodiments, the stent comprises a biodegradable material whereby the method includes permitting the stent to be at least partially absorbed by the subject.

In some embodiments, securing the stent to the abdominal wall includes contracting the stent to move toward a compressed state to facilitate introduction within the opening in the abdominal wall and transitioning the stent toward an expanded state whereby the stent body engages tissue portions defining the opening in the abdominal wall. In other embodiments, the stent is normally biased toward its expanded state and wherein transitioning the stent includes releasing the stent from its compressed state to permit the stent to move toward its expanded state. In embodiments, securing the stent to the abdominal wall includes expanding the stent with an expansion member introduced within a longitudinal opening of the stent body.

The stent and its application in an ostomy procedure will maintain the patency of the stoma while supporting the tissue including cutaneous, fascia and muscle tissue (e.g., the anterior rectus sheath) surrounding the opening during healing to minimize the potential of complications, particularly, the formation of a parastomal hernia. The stent may be implanted to encompass one, two or all layers of fascia depending on the anatomy of the subject. The end flanges with anchors secure the stent within the opening with minimal potential for dislodgment during the healing process. Generally stated, the stoma established with the stent minimizes the issues associated with conventional suture created stoma methodologies including necrosis, dislodgement, prolapse, stenosis and mucocutaneous separation.

Other features of the present disclosure will be appreciated from the following description.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description given below, serve to explain the principles of the disclosure, wherein:
FIG. 1 is a schematic illustration of an abdominal region of a subject with a surgically created stoma;
FIG. 2 is a perspective view of the stent for supporting a stoma within abdominal tissue illustrating the stent body, the end flanges and anchor members;
FIG. 3 is a cross-sectional view of the stent taken along the lines 3-3 of FIG. 2; and
FIG. 4-11 are views illustrating a sequence of use of the stent in formation of a stoma in connection with a colostomy procedure.

### DETAILED DESCRIPTION

Particular embodiments of the present disclosure are described hereinbelow with reference to the accompanying drawings. However, it is to be understood that the disclosed embodiments are merely examples of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to employ the present disclosure in virtually any appropriately detailed structure.

The following discussion of the surgical stent will focus on in its application in facilitating formation of a stoma in an ostomy procedure, particularly, in securing the end margins of an intestine to the abdominal tissue in connection with a colostomy or ileostomy procedure. However, the stent has application in other ostomy procedures including ileostomy, urostomy, gastrostomy and jejunostomy procedures.

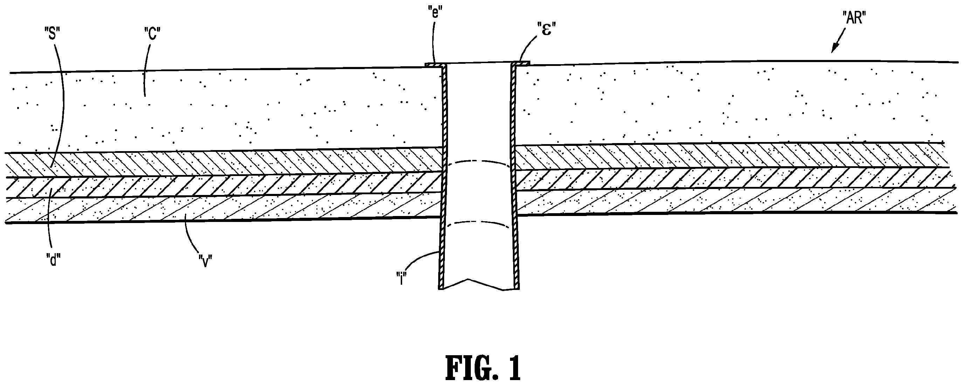

Referring to FIG. 1, an abdominal region "AR" of a subject's body generally includes abdominal tissue having an outer cutaneous layer "c" (e.g., epidermis, dermis, and hypodermis) and three fascia layers (e.g., superficial fascia "s", deep fascia "d" and visceral fascia "v") that enshroud muscles including the rectus sheath, organs, vessels, and/or other tissue for performing various bodily functions such as digestion. For instance, as the part of a digestive system of a subject's body, the stomach and the intestines are supported in the abdominal region "AR". In the course of a natural digestion process, the stomach and the intestines collaborate with the rest of the digestive system to process food and excrete fecal matter through the anus. Unfortunately, as a result of disease or injury to the intestines, it may become necessary to bypass natural digestion by providing an artificial stoma through the abdominal region "AR" in order to safely excrete the fecal matter from the subject's body. To create the stoma, the end margins of the intestine "i" or colon are advanced through an opening in the abdomen formed via a scalpel or the like and at least partially flared outwardly to circumscribe the opening. In a conventional ostomy procedure, the end margins "e" would be sutured to the cutaneous layer "c" and the underlying fascia layers "s", "d", "v" to create the stoma and effect the colostomy. As discussed hereinabove, due to various factors, there exists a potential of the formation of a parastomal hernia adjacent the dissected area of fascia and muscle.

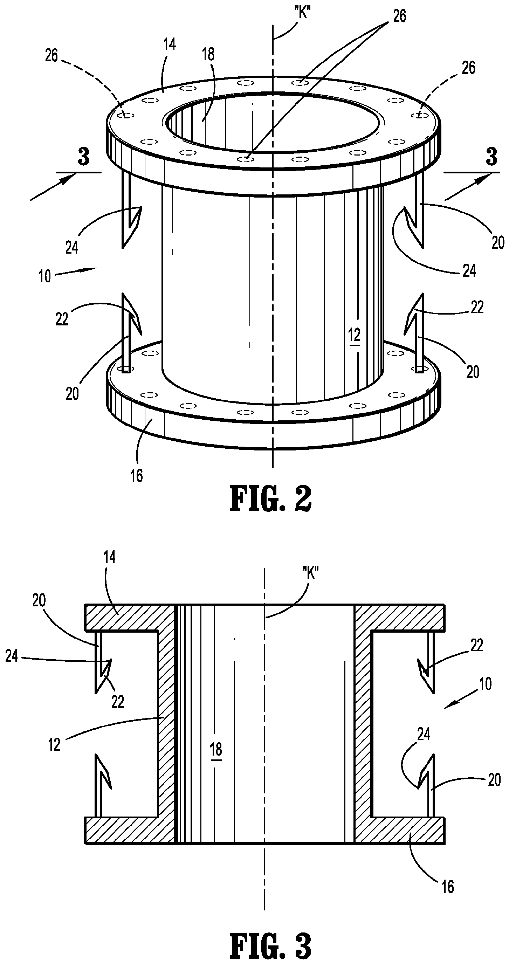

Referring now to FIGS. 2-3, there is illustrated the stent 10 for placement within the stoma to stabilize the stoma and surrounding areas, and to minimize the potential of the formation of a parastomal hernia. The stent 10 includes a stent body 12 defining a longitudinal axis "k" and having opposed end flanges 14, 16 at respective ends of the stent body 12. The stent body 12 may define a longitudinal opening 18 to permit placement over a stent delivery instrument such as a mandrel or a balloon catheter. The stent body 12 may be fabricated from any biocompatible material including spring steel, stainless steel, a shape memory alloy, or super elastic alloy, e.g. Nitinol, a Co-Cr alloy, a biodegradable or bioabsorbable polymer including synthetic and naturally derived polymers and combinations thereof. Examples of suitable polymeric materials include polylactide, polyglycolide, poly-L-lactide, poly-D-lactite, polylactic acid, etc. The polymers of the biodegradable stents may be selected to degrade over a predetermined time period sufficient to permit the stoma to stabilize to minimize the potential of herniation. The stent body 12 may be impregnated or coated with a therapeutic agent or may be lined with either an inner or outer sleeve (such as polyester fabric or ePTFE) for tissue ingrowth. The stent body 10 may be a drug eluting stent capable of releasing antibacterials, antimicrobials, etc. The stent body 12 can be any size, shape, internal structure, etc., and include braided stents or other forms of stents such as a laser-cut stents, knitted stents, and the like, as long as the stent is expandable within the lumen of the intestine.

In embodiments, the stent body 12, whether formed of a metallic material or a polymeric material, exhibits sufficient resiliency or elasticity such that the stent body 12 can be compressed to achieve a compressed state and then return to its normal expanded state when released. In other embodiments, the stent body 12 may be inelastically deformable to transition from a normal unexpanded state to an expanded state with the use of a stent expansion or deployment instrument such as disclosed in commonly assigned U.S. Patent No. 6,589,274 to Stiger, et al.

Each end flange 14, 16 may be fabricated from the same material as the stent body 12, e.g., be monolithically formed with the stent body 12. In the alternative, the end flanges 14, 16 may be fabricated from a different material than the stent body 12 and coupled to the stent body 12 via conventional methodologies, including cements, adhesives, welding or the like. Each end flange 14, 16 has a cross-sectional dimension orthogonal to the longitudinal axis "k" greater than a corresponding cross-sectional dimension of the stent body 12. Each end flange 14, 16 has at least one, preferably, two diametrically opposed anchors 20 which depend from the flanges 14, 16 and extend along the longitudinal axis "k". More than two anchors 20 are also envisioned. The anchors 20 may include barbs 22 having pointed ends 24 to assist in penetration through fascia and/or muscle tissue to facilitate attachment of the flanges 14, 16 to the tissue. Each end flange 14, 16 also may include a plurality of spaced flange openings 26 extending through the respective flanges 14, 16 for reception of sutures or staples to further assist in securing the end flanges 14, 16 relative to the fascia and muscle tissue. Each end flange 14, 16 may deflect or pivot about the area or juncture of attachment with the stent body 12 (e.g., similar to a living hinge) to facilitate insertion of the end flanges 14, 16 and the stent body 12 within the tissue. Although shown as similar in configuration and dimension, it is appreciated that the end flanges 14, 16 may differ in configuration relative to each other depending on the use of the stent 10 and the subject's anatomy.

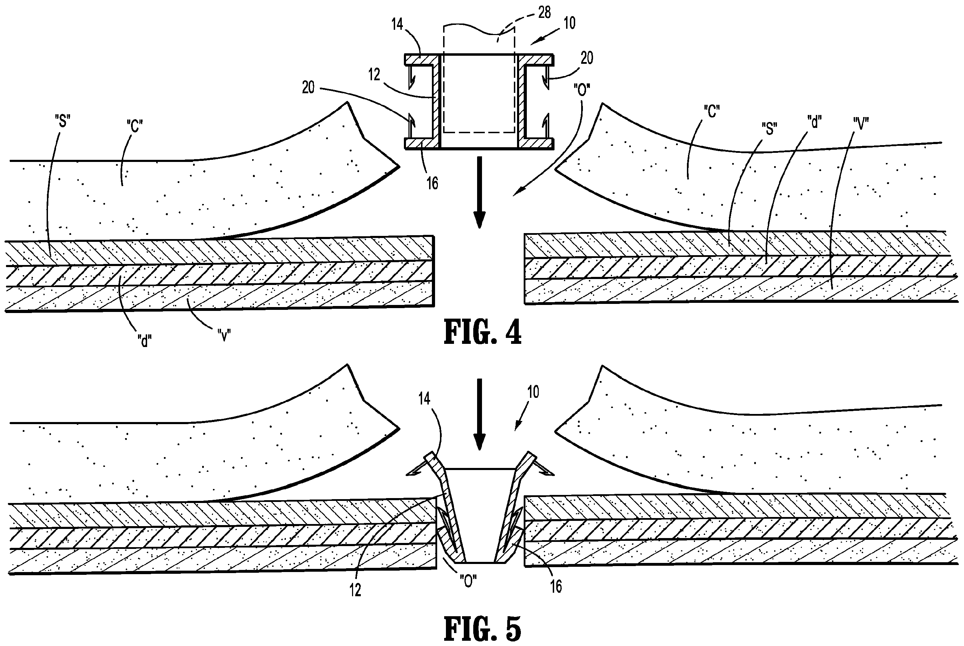

FIGS. 4-11 are views illustrating a sequence of use of the stent 10 in a colostomy procedure. With initial reference to FIG. 4, an opening or incision "o" is made through the abdominal wall, e.g., extending through the cutaneous layer "c" and the underlying fascia layers including the superficial fascia "s", deep fascia "d" and visceral fascia "v", to communicate with the abdominal cavity "a". Thereafter, the stent 10 is introduced within the opening "o" either manually or with the use of a stent insertion mandrel or instrument identified schematically as instrument 28. Suitable stent insertion instruments are disclosed in commonly assigned U.S. Patent Publication No. 20070288080 to MacCollum et al, the entire contents of which are hereby incorporated by reference herein. During insertion, at least one of the flanges 14, 16 may deflect to facilitate advancement through the tissue defining the opening "o". (FIG. 5) In addition, the stent body 12 may be compressed to a compressed state to assume a reduced cross-sectional dimension to further assist in passing the stent body 12 through the abdominal opening "o".

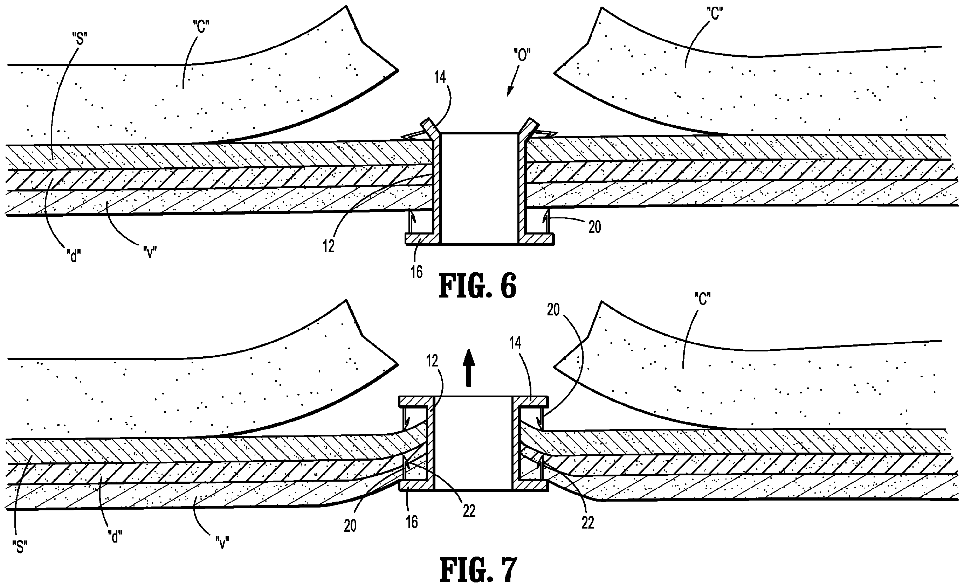

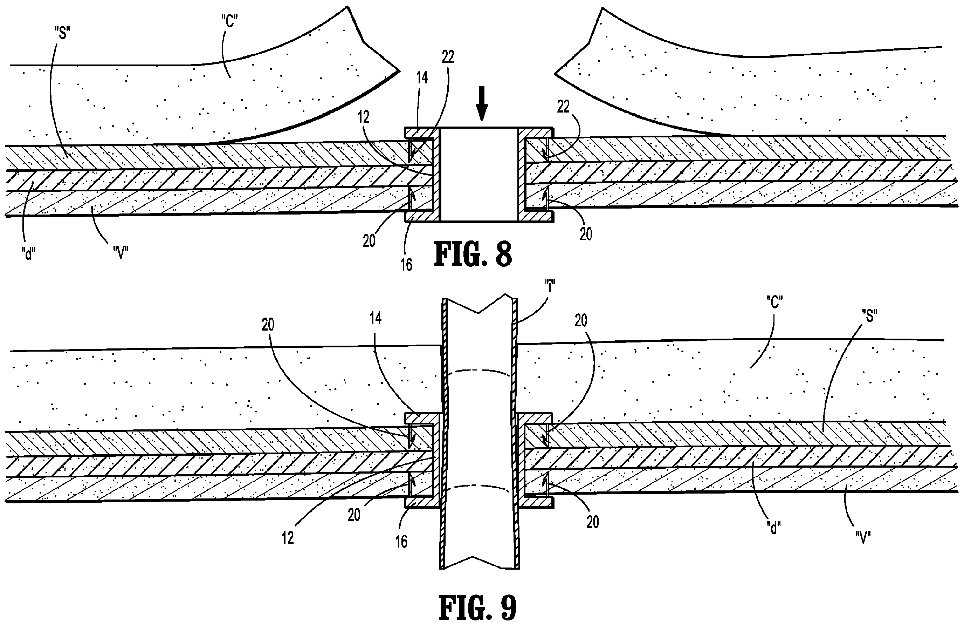

With references to FIGS. 6-8, the stent 10 is released to assume its normal expanded state in engagement with tissue defining the opening "o" in the abdominal wall. The stent 10 is secured relative to the cutaneous layer "c" and fascia layers "s", "d", "v" through, e.g., retracting (FIG. 7) and advancing (FIG. 8) movement of the stent body 12 relative to the fascia layers "s", "d", "v" whereby the anchors 20 and barbs 22 penetrate and secure the end flanges 14, 16 relative to the tissue. In embodiments, only the superficial fascia "s" is secured between the end flanges 14, 16. In other methodologies, the superficial fascia "s" and the deep fascia "d" are encompassed by the stent body 12. In other embodiments, each of the superficial fascia "s", deep fascia "d" and visceral fascia "v" are engaged by the end flanges 14, 16 of the stent 10. Other combinations are also envisioned. With the stent 10 positioned relative to the opening "o", the clinician may optionally secure the end flanges 14, 16 to the one or more of the fascia layers "s", "d", "v" with the use of sutures or fasteners including staples, tacks or clips which are passed through the flange openings 26 in the end flanges 14, 16. (FIG. 2)

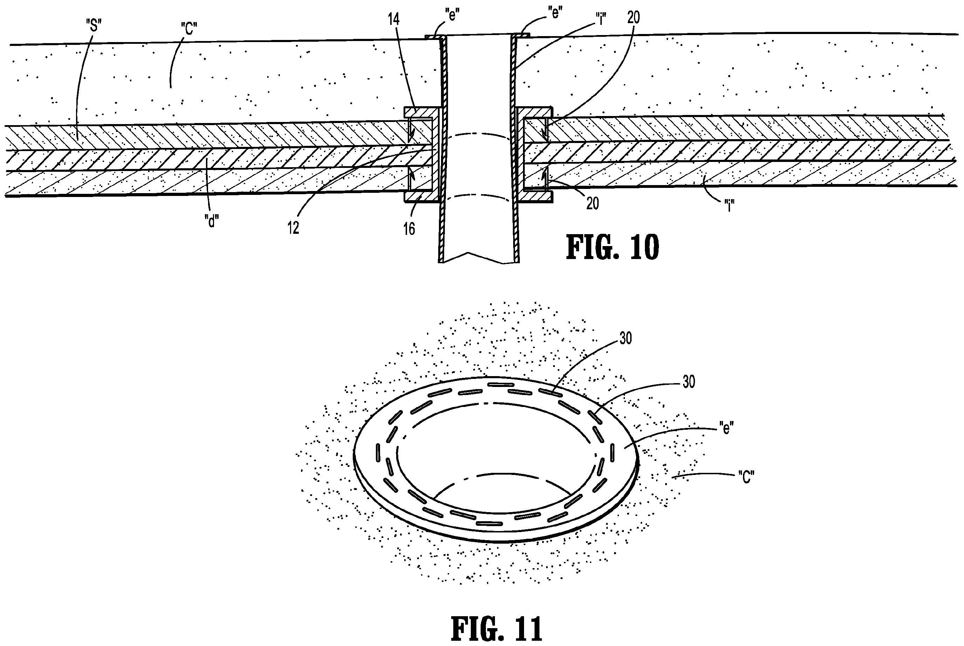

Once the stent 10 is secured, attention is directed to creating the stoma. With reference to FIG. 9, the intestinal section "i", e.g., a colon section, is resected and passed through the longitudinal opening 18 of the stent body 12. Thereafter, the end margins "e" of the intestinal section "i" are folded back toward the cutaneous layer "c" as depicted in FIG. 10 to complete the stoma. The end margins "e" of the intestinal section "i" may be secured to the cutaneous layer "c" of the abdominal wall with the use of sutures or other fasteners 30 as shown in FIG. 11. The fasteners 30 may be in the form of a surgical staple, clip, tack or pin, and may be bioabsorbable.

The stent 10 will support the tissue surrounding the stoma during the healing process to minimize the potential of tissue migration and formation of a parastomal hernia. The stent 10 may be removed upon healing of the digestive system, or, if formed of a biodegradable material, will degrade and be absorbed by the body of the subject over a predetermined period of time.

The above description and the drawings are provided for the purpose of describing embodiments of the present disclosure and are not intended to limit the scope of the disclosure in any way. It will be apparent to those skilled in the art that various modifications and variations can be made without departing from the spirit or scope of the disclosure. Thus, it is intended that the present disclosure cover the modifications and variations of this disclosure provided they come within the scope of the appended claims and their equivalents.

The invention may be described by reference to the following numbered paragraphs:-
1. A method for facilitating formation of a stoma, comprising:
   positioning a stent within an opening extending through an abdominal wall of a subject;
   accessing an internal intestinal segment through the opening in the abdominal wall;
   passing the intestinal segment through the stent and external of the abdominal wall; and
   securing end margins of the intestinal segment against the abdominal wall surrounding the opening.
2. The method according to paragraph 1 including securing the stent to the abdominal wall.
3. The method according to paragraph 2 wherein the stent includes a stent body and at least one end flange disposed at an end of the stent body and wherein securing the stent includes attaching the end flange to the abdominal wall.
4. The method according to paragraph 3 wherein the stent includes end flanges at each end of the stent body and wherein securing the stent includes attaching each end flange to the abdominal wall.
5. The method according to paragraph 4 wherein the end flanges each include anchors and wherein securing the stent includes penetrating the abdominal wall with the anchors of the end flanges.
6. The method according to paragraph 5 wherein securing the stent includes penetrating at least one of the superficial fascia, deep fascia and visceral fascia with the anchors of the end flanges.
7. The method according to paragraph 6 wherein securing the stent includes penetrating at least two of the superficial fascia, deep fascia and visceral fascia with the anchors of the end flanges.
8. The method according to paragraph 1 including forming an incision within the abdominal wall, the incision being the opening.
9. The method according to paragraph 1 wherein the stent comprises a biodegradable material and including permitting the stent to be at least partially absorbed by the subject.
10. The method according to paragraph 2 wherein securing the stent to the abdominal wall includes contracting the stent to move toward a compressed state to facilitate introduction within the opening in the abdominal wall and transitioning the stent toward an expanded state whereby the stent body engages tissue portions defining the opening in the abdominal wall.
11. The method according to paragraph 10 wherein the stent is normally biased toward its expanded state and wherein transitioning the stent includes releasing the stent from its compressed state to permit the stent to move toward its expanded state.
12. The method according to paragraph 2 wherein securing the stent to the abdominal wall includes expanding the stent with an expansion member introduced within a longitudinal opening of the stent body.

## Claims

1. A stent (10) for use in the formation of a stoma, said stent (10) comprising:
a stent body (12) defining a longitudinal axis ("k") and preferably including a longitudinal opening, at least one end flange (14,16) disposed at an end of the stent body (12) and wherein the end flange (14,16) is configured for attachment to the abdominal wall.

2. The stent (10) according to claim 1, further comprising end flanges (14, 16) at each end of the stent body (12) and wherein said end flanges (14, 16) are configured for attaching to the abdominal wall.

3. The stent (10) according to claim 2, wherein the end flanges (14, 16) each include anchors (20) configured to penetrate the abdominal wall.

4. The stent (10) according to claim 3 wherein the anchors (20) are configured to penetrate at least one of the superficial fascia, deep fascia and visceral fascia.

5. The stent (10) according to claim 3 wherein the anchors (20) are configured to penetrate at least two of the superficial fascia, deep fascia and visceral fascia.

6. The stent (10) according to any one of claims 3 to 5, wherein the anchors (20) comprise barbs.

7. The stent (10) according to any preceding claim wherein said stent (10) comprises a biodegradable material which allows the stent (10) to be at least partially absorbed during use.

8. The stent (10) according to any preceding claim, wherein said stent (10) is configured to transition between a compressed and an expanded state.

9. The stent (10) according to claim 8, wherein the stent (10) is normally biased toward its expanded state and wherein transitioning the stent (10) includes releasing the stent from its compressed state to permit the stent to move toward its expanded state.

10. The stent (10) according to any preceding claim, wherein each end flange (14, 16) has a cross-sectional dimension orthogonal to the longitudinal axis "k" greater than a corresponding cross-sectional dimension of the stent body (12).

11. The stent (10) according to any preceding claim, wherein each end flange (14, 16) is configured to deflect or pivot about the area or juncture of attachment with the stent body (12).

12. The stent (10) according to any preceding claim, wherein each end flange (14, 16) includes a plurality of spaced flange openings (26) extending through the respective flanges (14, 16) for the reception of sutures or staples.

13. The stent (10) according to any preceding claim, wherein the stent body (12) is impregnated or coated with a therapeutic agent or is drug eluting.

14. The stent (10) according to any preceding claim, wherein the stent body (12) is lined with either an inner or outer sleeve, preferably comprising polyester fabric or ePTFE.
